# FASCICULE DE BREVET EUROPEEN

(11) **EP 3 116 419 B1**
(45) Date de publication et mention de la délivrance du brevet: **27.12.2017**
(21) Numéro de dépôt: 15709696.7
(22) Date de dépôt: 16.03.2015
(51) Int. Cl.: A61B 17/54, A61B 17/32, A61B 17/00

(54) **DISPOSITIF POUR LA MICRODERMABRASION ET PROCÉDÉ DE MICRODERMABRASION**
MIKRODERMABRASIONSVORRICHTUNG UND MIKRODERMABRASIONSVERFAHREN
MICRODERMABRASION DEVICE AND MICRODERMABRASION METHOD

(30) Priorité: 14.03.2014 FR 1452114
(43) Date de publication de la demande: 18.01.2017
(73) Titulaire: Favie, Alain, 29200 Brest (FR); Boloorchi, Armand, 29200 Brest (FR)
(72) Inventeur: Favie, Alain, 29200 Brest (FR); Boloorchi, Armand, 29200 Brest (FR)
(74) Mandataire: Ipside
(86) Numéro de dépôt international: PCT/EP2015/055383
(87) Numéro de publication internationale: WO 2015/136116

(56) Documents cités:
- EP-A1- 0 381 962
- WO-A1-2014/169237
- AU-B2- 2011 265 418
- US-A1- 2008 103 563
- US-A1- 2010 326 456

## Description

### Domaine technique et état de l'art

La présente invention se rapporte à un dispositif pour la microdermabrasion de la peau dans le domaine des soins esthétiques, à destination des professionnels des soins esthétiques, notamment non-médecins.

La microdermabrasion est un procédé consistant de retirer une couche très superficielle de la peau, constituée de cellules mortes. La microdermabrasion permet également de relisser la peau. La microdermabrasion unifie le teint et rend la peau plus éclatante en éliminant les cellules mortes et favorisant le processus naturel de réparation des tissus. La microdermabrasion est principalement utilisée sur le visage, le dos des mains, le décolleté, les vergetures des seins, les vergetures du ventre, les vergetures des hanches et les vergetures des fesses, mais aussi d'autres zones tels que les bras ou les cuisses notamment pour améliorer le grain de peau, atténuer des imperfections ou densifier et retendre la peau.

La microdermabrasion élimine, outre la couche cornée et les cellules mortes, la couche superficielle de l'épithélium, tout en restant à distance de la membrane basale de l'épithélium.

Le document WO 2006/045149 décrit un dispositif pour l'abrasion cutanée comprenant un corps vibrant et un embout interchangeable comportant une surface abrasive. Il permet de réduire la couche d'épiderme pour créer une peau neuve. D'autres exemples décrivent un dispositif pour l'abrasion comme le document EP 0 391 962, AU 2011 264 418 (le préambule de la revendication 1 est basé sur ce document) et US 2008/103563.

Toutefois, ces dispositifs sont :
- difficile à manier,
- ne permettent pas d'assurer une bonne hygiène d'utilisation,
- n'assurent pas un bon contact entre la surface abrasive et la peau.

### Description de l'invention

L'invention vise à remédier aux inconvénients de l'art antérieur et concerne à cette fin un dispositif pour la microdermabrasion comprenant :
- un embout détachable (11) comprenant au moins un élément abrasif (112), ledit élément abrasif (112) étant prévu pour être appliqué sur la surface de la peau,
- un corps comportant un moyen vibrant, ledit moyen vibrant étant adapté pour la mise en vibration du corps ou de l'embout détachable (11),
- un support d'embout (12) fixé au corps, remarquable en ce que l'embout détachable (11) comprend un élément déformable (113) positionné entre l'élément abrasif (112) et le support d'embout (12), ledit embout détachable (11) comprend un élément protecteur (111) positionné à l'extrémité de l'embout détachable et ledit élément protecteur (111) étant de la forme d'une demi-sphère ou d'une demi-sphère aplatie.

Le dispositif permet d'améliorer le confort d'utilisation sur la peau et est adapté à l'utilisation pour toutes les zones du corps. L'élément déformable effleure la peau lors de l'utilisation du dispositif objet de l'invention. De plus, l'élément déformable permet l'adaptation de l'élément abrasif à la forme de la peau grâce à sa déformation sous la pression. L'élément protecteur permet de protéger la surface de la peau du bord de l'élément abrasif.

Les termes « élément déformable » désignent un élément constitué d'une matière déformable qui reprend sa forme d'origine lorsque la contrainte qu'on lui applique disparaît.

L'invention est avantageusement mise en oeuvre selon les modes de réalisation exposés ci-après. Les modes de réalisation sont à considérer à titre individuel ou selon toutes combinaisons techniquement opérantes.

Selon un aspect de l'invention, l'élément déformable est en élastomère dont la dureté SHORE est comprise entre 10 et 30. La dureté SHORE est définie par les normes ISO 868 et 7619 sur une échelle allant de 0 à 100, de mou à dur. La dureté est en fonction de l'élasticité du matériau.

Par sécurité, la dureté est choisie pour qu'une pression trop importante stoppe la vibration et n'a aucun effet sur la peau.

Selon un autre aspect de l'invention, dans un plan de coupe au dispositif contenant l'axe longitudinal du corps et l'axe longitudinal du support d'embout, l'axe longitudinal du support d'embout et l'axe longitudinal du corps forment un angle compris entre 90° et 270°, de préférence, de 135°. Cet angle entre l'embout détachable et le corps augmente la maniabilité de l'objet de l'invention et le confort de l'utilisateur.

Selon un autre mode de réalisation, le dispositif comprend une liaison pivot entre le support d'embout et le corps du dispositif, l'axe de ladite liaison pivot étant contenu dans un plan orthogonal à l'axe longitudinal du corps et passant par un point d'intersection de l'axe longitudinal du support d'embout avec l'axe longitudinal du corps.

La liaison pivot oriente l'embout détachable par rapport au corps et adapte le dispositif aux différentes prises en main et surfaces de la peau.

Avantageusement, le corps de l'élément déformable est de forme ovale, triangulaire, polygonale d'au moins cinq côtés ou d'un cercle, dont son centre est troué pour s'adapter au support d'embout.

Selon une variante du dispositif objet de l'invention, le moyen vibrant comprend un moteur entraînant en rotation un élément comportant un balourd.

Selon cette dernière variante, le dispositif objet de l'invention comprend des moyens pour faire varier la vitesse de rotation du moteur entre 0 tr/min et 12 000 tr/min.

Selon une autre variante, le dispositif comprend au moins des moyens d'éclairage, notamment une diode électroluminescente dont la longueur d'onde est comprise entre 400 et 900 nm et entre sa fluence comprise entre 3 et 4 J/cm².

Selon cette dernière variante, la longueur d'onde est comprise entre 570 et 630 nm.

Le terme DEL (Diode électroluminescente) désigne un composant optoélectronique capable d'émettre de la lumière lorsqu'il est parcouru par un courant électrique.

La fluence (par exemple, 3,4J/cm²) assure une bonne efficacité de l'application de la diode électroluminescente.

Selon une autre variante, l'élément déformable comprend une cavité agencée pour recevoir un volume d'une matière crémeuse, tel que de la crème adoucissante.

Ainsi, lors de l'utilisation, la cavité est comprimée ce qui fait sortir la crème.

Selon une autre variante de la présente invention, le dispositif comprend des moyens d'étanchéité. De cette manière, le dispositif est nettoyable en le passant sous l'eau.

Selon une autre variante de la présente invention, l'élément abrasif comprend un moyen de fixation, tel que de la colle ou du velcro, agencé pour maintenir en position l'élément abrasif sur l'élément déformable.

Également selon une variante, l'élément abrasif est un ruban à usage unique, agencé pour être positionné autour de l'élément déformable et une partie dudit ruban est détachable pour permettre le retrait de l'élément abrasif.

Selon des modes de réalisation, l'embout détachable est mobile en rotation autour de l'axe longitudinal du support d'embout.

Selon des modes de réalisation, l'embout détachable est entraîné en rotation par un moyen rotatif. Le moyen rotatif est un moteur dont son axe de rotation est relié directement ou indirectement (par exemple l'axe du moteur est relié à un pignon conique à l'embout détachable).

L'invention concerne également, un procédé de microdermabrasion pour des soins esthétiques non thérapeutique comprenant les étapes suivantes :
- a) mise en place d'un embout détachable sur le dispositif objet de l'invention,
- b) mise en vibration de l'élément abrasif sur la surface de la peau, pendant une durée de 1 à 30 minutes pour une surface de l'ordre de 50 à 300 cm².

Selon une variante, le procédé comprend une étape c), après l'étape b), enlèvement de l'élément abrasif et application d'une crème adoucissante sur la surface de la peau pendant une durée de 2 à 20 minutes.

Grâce à ce dispositif, on constate dès la première séance une amélioration du grain de peau et un meilleur éclat pour le visage. En effet, le grain de peau est immédiatement affiné et le coup d'éclat est instantané. Avec un nombre suffisant de séances (entre 5 et 10 séances en phase de démarrage et des séances régulières de suivi) les résultats sont comparables, et même meilleurs qu'avec des moyens chimiques (peeling) ou physiques existants (laser).

Au fil des séances de microdermabrasion, associée aux massages et une onde lumineuse, pour le visage, les fines ridules s'aplanissent et les défauts de surface de la peau sont atténués. La peau paraît plus jeune, plus lisse et uniforme. Également au niveau du corps où la peau apparaît plus, jeune, plus dense, et plus tonique.

Les effets à long terme, si l'utilisation est récurrente, sont :
- une amélioration du grain de peau,
- une augmentation du taux de collagène et des cellules de l'épithélium, donc de la souplesse, de l'épaisseur et de la qualité de la peau.

D'une façon générale, l'invention permet de ralentir les effets du temps sur la peau, en profondeur et de conserver une peau de surface toujours « neuve ». Elle permet de ralentir et d'atténuer l'apparition d'imperfections sur la peau, liées au temps, grâce au lissage de la peau qui est fait.

Selon une autre variante, le procédé comprend une étape d) préalable à l'étape a), de retrait de l'élément abrasif d'un sac étanche et de la mise en place dudit élément abrasif sur l'embout détachable. De cette manière, l'élément abrasif est à usage unique ce qui garantit une hygiène d'utilisation à chaque fois.

### Brève description des figures

D'autres caractéristiques et avantages de l'invention apparaîtront à la lumière de la description qui suit, réalisée sur la base des dessins annexés. Ces exemples sont donnés à titre non limitatif. La description est à lire en relation avec les dessins annexés dans lesquels :
- la figure 1 représente un exemple de réalisation de l'invention selon une vue en perspective,
- la figure 2 représente une vue éclatée d'un exemple de réalisation du dispositif objet de l'invention,
- les figures 3 et 4 représentent une vue de détail sur l'embout détachable du dispositif selon un exemple de réalisation d'invention,
- les figures 5 à 7 représentent comment retirer la partie abrasive de l'embout détachable,
- la figure 8 représente un autre exemple de réalisation de l'invention selon la figure 5,
- les figures 9 et 10 représentent différentes formes de l'embout détachable,
- la figure 11 représente un exemple d'invention du corps du dispositif,
- la figure 12 représente une vue de côté pour montrer l'angle entre l'embout détachable et le corps du dispositif,
- les figures 13 et 14 représentent une vue d'un autre exemple de l'invention,
- la figure 15 représente une vue éclatée du moyen vibrant,
- la figure 16 représente une vue de côté d'un autre exemple du dispositif avec de l'air pulsé,
- la figure 17 représente une vue en perspective de la sortie de l'air pulsé,
- la figure 18 représente un autre exemple de l'invention,
- la figure 19 représente une vue éclatée du dispositif selon un exemple de l'invention,
- la figure 20 représente une vue de détail sur le guide câble du dispositif selon un exemple de l'invention,
- la figure 21 représente une vue de détail sur le moyen de connexion de la base,
- les figures 22 et 23 représentent une vue de côté d'un exemple du dispositif avec deux lumières,
- la figure 24 représente une coupe histologique de la peau d'un patient avant l'application du dispositif,
- la figure 25 représente une coupe histologique de la peau d'un patient après quatre séances d'application du dispositif.

### Description de modes de réalisation de l'invention

Le dispositif pour la microdermabrasion a un double usage : microdermabrasion et massage.

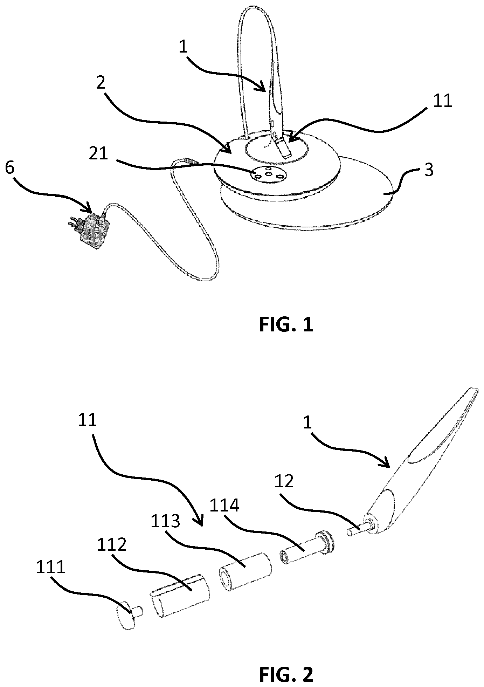

A la figure 1, il est montré le dispositif 1 pour la microdermabrasion montée sur sa base 2. Le dispositif 1 comprend un embout détachable 11 Il est montré sur cette figure l'alimentation externe (transformateur 6) au dispositif 1 qui vient alimenter le dispositif ou un support au dispositif. Cette figure représente également une interface 21 qui permet de commander le dispositif 1 et notamment de faire varier la vibration.

Dans un exemple de réalisation, l'embout détachable 11 est de forme allongée.

Le transformateur 6 (alimentation externe) peut être relié à la base, au support ou au dispositif 1 (cette variante sera décrite ci-dessous).

Selon un autre exemple d'un mode de réalisation, le dispositif comprend :
- soit une alimentation interne, telle qu'une batterie, et une commande sélectionnée parmi la mise en marche ou l'arrêt du fonctionnement du moteur, un variateur de vitesse du moteur ou une commande avec une vitesse prédéfinie,
- soit une base agencée pour recevoir le dispositif, ladite base comprenant une interface sélectionnée parmi la mise en marche ou l'arrêt du fonctionnement du moteur, un variateur de vitesse du moteur ou une commande avec une vitesse prédéfinie.

Selon un autre exemple, ledit dispositif comprend soit une alimentation externe reliée au dispositif ou à la base.

Également, selon un autre exemple, la base est agencée pour être fixée à un support par un moyen de connexion. Le moyen de connexion transmet de l'électricité à base, de sorte à alimenter électriquement le dispositif, le support comprenant une alimentation électrique. Le moyen de connexion permet également de fixer la base au support.

A la figure 2, il est montré un embout détachable 11 comprenant plusieurs éléments. A une extrémité de l'embout détachable 11, il est placé l'embout de protection 111. Un élément abrasif 112 permet d'utiliser le dispositif pour la microdermabrasion. L'élément abrasif 112 est adapté pour être appliqué sur la surface de la peau.

L'élément abrasif 112 est du papier abrasif dont le grain est compris entre 100 et 600 grains par mètres carrés. L'élément abrasif 112 se positionne autour d'un élément déformable 113.

Dans un autre mode de réalisation, l'élément abrasif 112 comprend un moyen de fixation, tel que de la colle ou du velcro. Ce moyen de fixation est adapté pour maintenir en position l'élément abrasif 112 sur l'élément déformable 113.

L'élément déformable 113 est par exemple en élastomère. Cet élément déformable 113 est optimisé avec une densité mesurée, pour rendre la phase de microdermabrasion et de massage optimum et confortable. Selon l'échelle de dureté Shore, la mesure de l'élastomère est comprise entre 10 et 30, de préférence 18.

Dans un autre exemple de l'invention, l'élément déformable 113 possède une crème contenue dans des cavités. Ces cavités permettent de recevoir un certain volume d'une crème, telle que de la crème régénératrice, apaisante, astringente...

Sur la figure 2, il est montré également le centre rigide 114 qui est adapté pour se positionner sur le support d'embout 12. L'embout détachable 11 se positionne sur un support d'embout 12 du dispositif 1. Le centre rigide 114 est évidé en son centre de la forme du support d'embout 12. Tel que représenté sur la figure 2, le support d'embout 12 est de forme cylindrique, donc le centre rigide à un alésage cylindrique. Selon une variante, le support d'embout 12 peut avoir la forme d'un carré ou posséder une surface plane pour mieux transmettre la vibration. Le centre rigide 114 a une forme inverse du support d'embout 12.

Il est possible également que le centre rigide 114 et l'élément déformable 113 soit bi-injecté de sorte à ne pas pourvoir dissocier le centre rigide 114 de l'élément déformable 113.

Dans un autre exemple de réalisation, l'élément protecteur 111 et l'élément déformable 113 sont solidaires l'une de l'autre.

L'élément protecteur 111 permet de protéger le patient de l'application de l'embout sur sa peau. Il évite également que le bord de l'élément abrasif 112 ne blesse le patient. Par exemple, l'élément protecteur 111 est de forme d'une demi-sphère ou d'une demi-sphère aplatie comme il est représenté sur la figure 2.

Dans un exemple de réalisation, l'élément protecteur est en position centrale sur l'embout. L'élément protecteur a un état de surface relativement lisse pour éviter de blesser le patient.

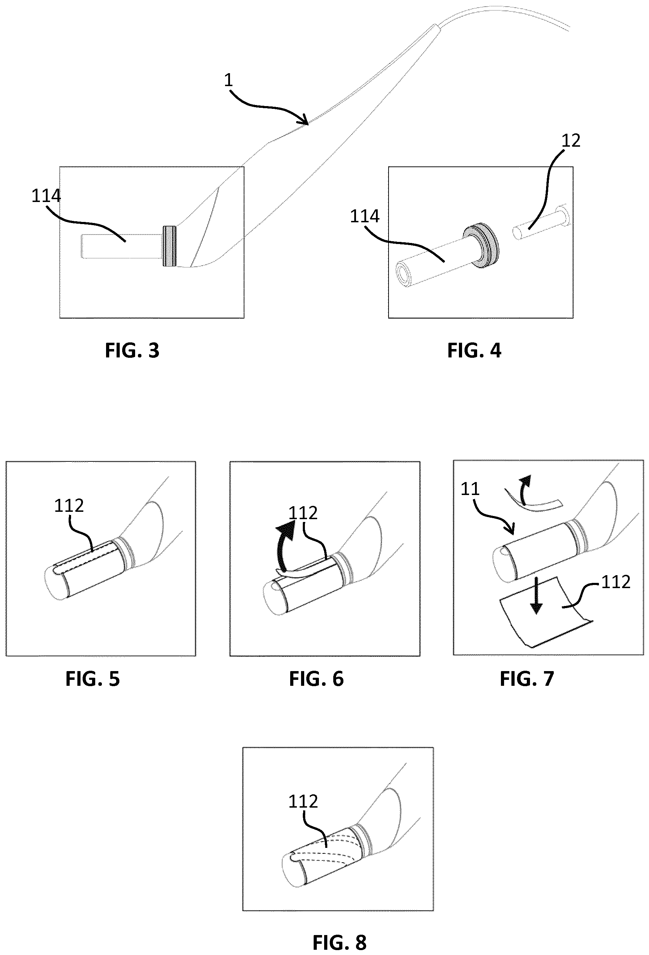

Les figures 3 et 4 représentent l'étape d'emboîtement de l'embout détachable sur le support d'embout 12. Il est montré le centre rigide 114. Le centre rigide 114 permet d'emboîter l'embout détachable 11 sur le support d'embout 12. Lors de l'emboîtement (non représenté), une bague ou un picot se casse pour empêcher la réutilisation. Cet emboîtement permet de fixer solidairement l'embout détachable 11 au dispositif 1, de sorte à bien transmettre les vibrations du dispositif 1 lors de son fonctionnement. Par exemple, l'embout détachable 11 est inséré et tourné pour entrer en liaison avec un bloqueur de position. Le fait de faire un tel mouvement, va engendrer un bruit pour avertir l'utilisateur de la mise en position et du maintien de l'embout détachable 11 au dispositif 1. De cette manière, il est garanti un usage unique pour assurer la qualité de l'embout en respectant l'hygiène et la sécurité.

Avant l'installation de l'embout détachable 11 sur le support d'embout 12. L'embout détachable 11 est contenu dans un sachet hermétique. De cette manière, l'hygiène est garantie jusqu'à l'ouverture du sachet.

Les figures 5 à 7 représentent une étape du retrait de l'élément abrasif 112. Par exemple, l'élément abrasif est un ruban interchangeable agencé pour être positionné autour de l'élément déformable 113. Le ruban est détachable pour permettre le retrait de l'élément abrasif. Par exemple, le ruban comprend une languette. La languette peut être sur toute sa longueur ou torsadée autour de l'élément abrasif 113, comme le montre la figure 8. Dans une variante, il n'y a que l'élément abrasif qui est changé, l'élément déformable 113 et le centre rigide 114 restent positionnés sur le support d'embout 12.

Dans un premier temps, il est nécessaire de repérer la languette. Dans un second temps, il est procédé au retrait de la languette pour retirer l'élément abrasif 112 de l'embout détachable 11. Le but de cette étape est de passer de l'étape de la microdermabrasion à l'étape de massage. L'étape de microdermabrasion est réalisée par l'élément abrasif 112 et l'étape de massage est réalisée par l'élément déformable 113. L'élément déformable 113 sert pour la phase de massage.

Selon une variante, les différents éléments du dispositif 1, comme l'embout détachable 11, sont réalisés par injection en bioplastique (plastique bio-sourcé, biodégradable, compostable...). De cette manière, le fait d'utiliser un embout détachage 11 (à usage unique) permet de les recycler.

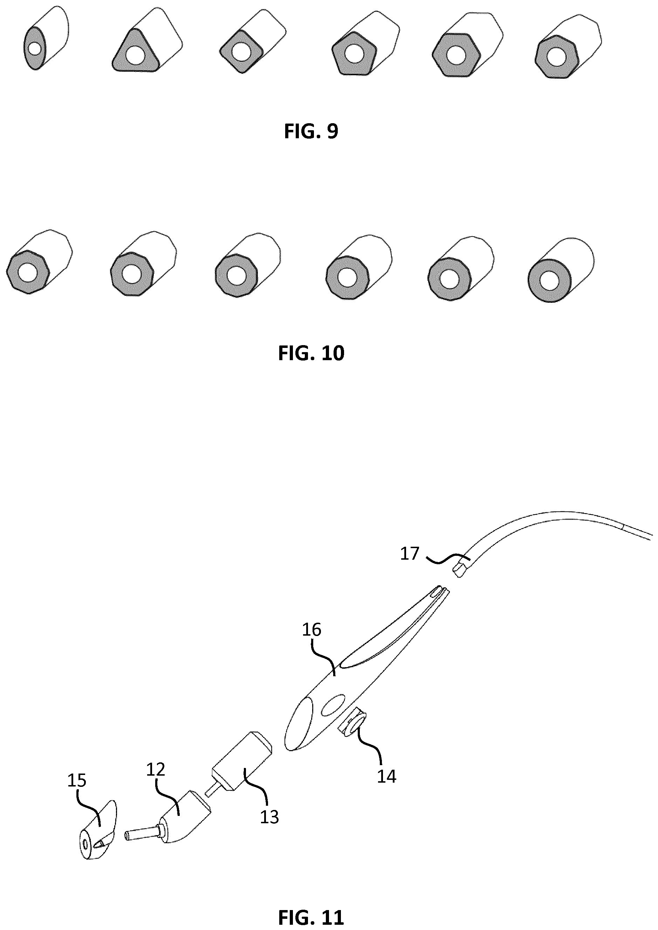

Les figures 9 et 10 représentent différentes formes de l'embout détachable 11 en perspective. L'élément déformable 113 monté solidairement au centre rigide 114 est de forme d'un ovale, d'un triangle, d'un carré, d'un polygone d'au moins cinq côtés ou d'un cercle. Sur ces figures il est montré que l'élément déformable est solidaire au centre rigide 114. De cette manière, la transmission des vibrations est assurée.

La figure 11 représente les différents éléments du dispositif 1. Il est représenté le support d'embout 12 qui coopère avec un moteur 13.

Le moteur 13 entraîne en rotation un moyen vibrant, tel qu'une masse excentrée par rapport à l'axe de rotation du moteur pour créer la vibration.

Dans un exemple de réalisation, un interrupteur 14 permet de faire varier la commande du moteur 13. Dans un autre exemple, l'interface 21 sur la base 2 peut faire varier la vitesse de rotation du moteur. Dans un exemple de réalisation, un interrupteur 14 permet de mettre en marche et arrêt le moteur. Un capot 14 vient recouvrir le support d'embout 12. Le capot 15 ne recouvre pas le support d'embout pour qu'il puisse recevoir l'embout détachable.

Dans une variante, le capot est muni d'une lumière, tel qu'une DEL, qui peut varier d'une lumière de l'ordre de 400 à 900 nm, de préférence de l'ordre de 570 à 630 nm. Dans une variante, une DEL est positionnée de chaque côté du capot 15. Dans une autre variante, le capot présente une DEL uniquement en dessous. Dans un autre exemple de réalisation, une DEL est positionnée au-dessus du capot 15.

Des essais cliniques utilisent des longueurs d'ondes suffisamment pénétrantes autour de 600nm ou dans l'infra rouge, des fluences (dose délivrée) de 1 à 20J/cm² et des irradiances (densité de puissance ou intensité) variant en fonction de la distance de la source lumineuse et de la taille du spot de 5 à 50mW/cm².

Les effets photo-biologiques dépendent du choix des paramètres de l'irradiation : la longueur d'onde, la dose délivrée (fluence), l'irradiance (densité de puissance ou intensité), le temps d'irradiation (durée d'exposition), le mode de délivrance continu ou pulsé avec des combinaisons possibles (temps de pulse-temps de pause) à l'infini pour ce dernier mode.

Dans un autre exemple de réalisation, les DEL éclairent le dessous et sur les côtés de l'embout détachable. Dans cet exemple, il n'y a pas de DEL dessus car l'embout détachage cacherait le faisceau lumineux.

Le dispositif utilise plusieurs longueurs d'ondes de la lumière.

Dans un exemple de réalisation, l'interface 21 ou le dispositif 1 a trois boutons pour sélectionner trois couleurs, comme le jaune à 588 nm, orange à 601 nm et rouge 628 nm.

Toujours à la figure 11, il est montré un surmoulage du câble 17 qui facilite la maniabilité du dispositif 1 en empêchant le câble 17 de retomber sur le dispositif 1. Le surmoulage permet d'avoir une forme arrondie du câble 17.

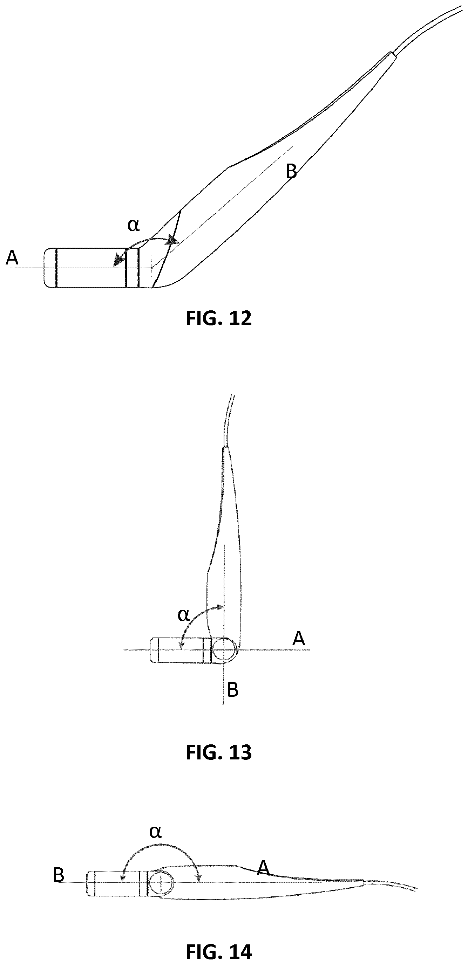

La figure 12 montre un angle α entre l'axe longitudinal A de l'embout détachable 11 et l'axe longitudinal B du dispositif 1.

L'axe longitudinal A de l'embout détachable 11 (comme montré sur la figure) est défini à l'intersection du « plan horizontal » de l'embout détachable 11 défini selon le sens habituel dans le référentiel terrestre et le « plan transversal » défini selon l'axe perpendiculaire au plan horizontal.

L'axe longitudinal B du dispositif 1 (comme montré sur la figure) est défini à l'intersection du « plan horizontal » du dispositif 1 défini selon le sens habituel dans le référentiel terrestre et le « plan transversal » défini selon l'axe perpendiculaire au plan horizontal.

Tel que montré à la figure 12, l'angle α est de 135°.

Dans une variante à l'invention, la figure 13 représente l'angle α à 90°. Dans la même variante, il est possible d'avoir un angle α à 270°. En effet, dans cette variante, l'angle α peut varier de 90° à 270°. Le dispositif 1 est relié à l'embout détachable 11 par un élément pivotant. Une liaison pivot est disposée entre le support d'embout 12 et le dispositif 1 (ou le corps du dispositif, tel que montré sur cette figure). La liaison pivot passe par un axe de rotation contenu dans un plan orthogonal à l'axe longitudinal du corps du dispositif 1 et passant par un point d'intersection de l'axe longitudinal du support d'embout et de l'axe longitudinal du corps.

Dans une variante, la liaison pivot est bloquée pour maintenir la position selon un angle α préféré. De cette manière, le dispositif permet de s'adapter aux zones de traitement où la peau est difficile d'accès.

Sur la figure 14, l'angle α est de 180°. Dans cette position, l'axe longitudinal A de l'embout détachable 11 et l'axe longitudinal B du dispositif 1 sont confondus.

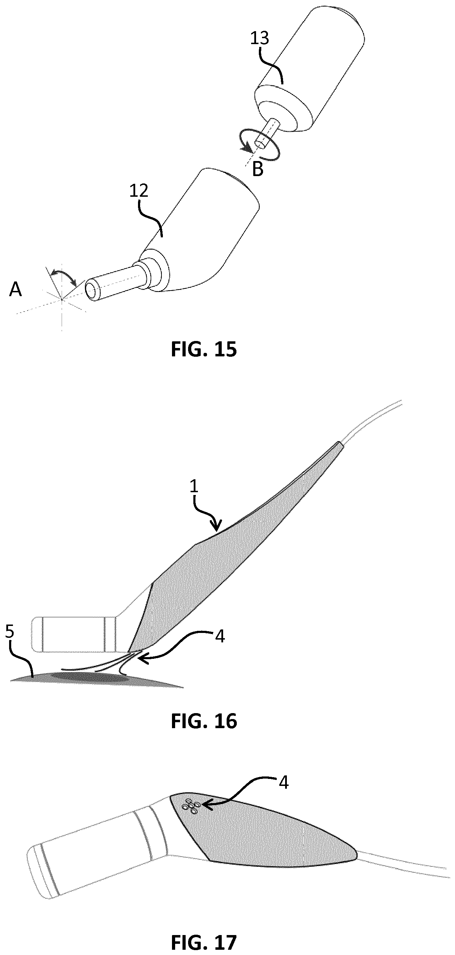

La figure 15 représente le moyen vibrant dans le support d'embout. Le moteur 13 est positionné selon l'axe longitudinal B du dispositif 1. Le moyen vibrant entraîne une oscillation du support d'embout 12 selon la fréquence de rotation du moteur 13.

Comme l'embout détachable 11 est solidaire du support d'embout 12, la mise en vibration du support d'embout 12 entraîne la vibration de l'embout détachable 11.

Comme précisé ci-dessus, le dispositif 1 ou la base 2 possède une commande de la mise en vibration (qui correspond à la mise en marche du moteur 13).

Lorsque le dispositif est muni d'un interrupteur 14, il peut agir sur le moyen vibrant (faire varier la vitesse du moteur) de 0 tr/min à 12 000tr/min. De préférence, il est prévu deux vitesses préprogrammées, une première vitesse de 6 000 tr/min et une deuxième vitesse de 3 000tr/min. La vitesse de 6 000 tr/min correspond à la phase de microdermabrasion et la vitesse de 3 000 tr/min correspond à la phase de massage. L'interrupteur permet également d'éteindre le dispositif.

Lorsque le dispositif n'a pas d'interrupteur 14, la commande peut se faire par l'interface 21. L'interface 21 possède les deux vitesses et/ou un variateur de vitesse.

Il est possible également d'avoir dans une autre variante, que toutes les commandes de l'interface 21 se retrouvent sur le dispositif 1. Ainsi, l'utilisateur peut choisir s'il utilise les commandes de l'interface 21 ou du dispositif.

Aux figures 16 et 17, il est représenté une variante du dispositif avec de l'air pulsé sur une zone de traitement 5. De cette façon, l'air chasse les peaux mortes de la zone de traitement 5 lors du fonctionnement de l'appareil. La figure 17 montre les sorties d'air 4.

Il est possible également d'avoir un dispositif étanche. De cette façon, le dispositif peut être nettoyé à l'eau. Des joints élastiques permettent de créer cette étanchéité et se retrouvent sur les différents éléments du dispositif 1, notamment de la coque.

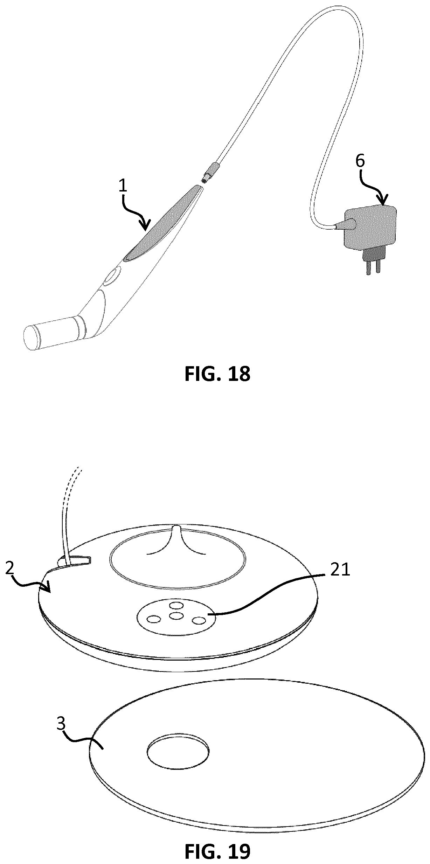

La figure 18 montre une autre variante de l'invention. Le dispositif 1, tel que montré, comprend une batterie interne qui peut être rechargée directement par un transformateur 6. La connexion se fait à une extrémité du dispositif 1 et permet de recharger et/ou d'alimenter le dispositif 1.

La figure 19 montre une vue éclatée du dispositif 1. Il est montré également l'interface 21 et la base 2. Cette figure montre également, un plateau d'accessoires 3. Le plateau d'accessoires est fixé par deux ou quatre vis sur la base 2.

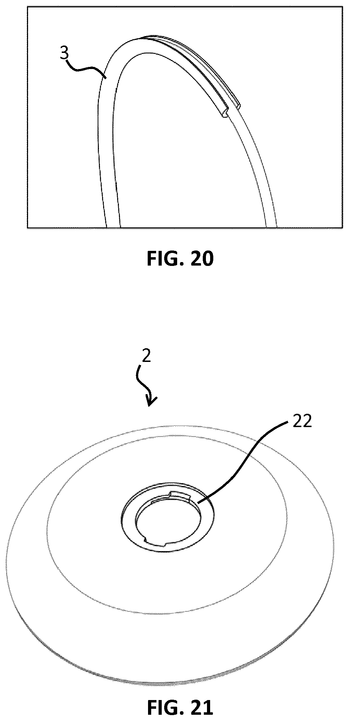

La figure 20 montre un autre exemple de réalisation d'invention dans lequel un câble 17 est positionné dans un guide câble. Le guide câble est cylindrique et comprend une gorge dans laquelle est positionné le câble 17.

La figure 21 montre le moyen de connexion 22. Le moyen de connexion 22 est de forme d'un disque et comprend des baïonnettes pour permettre un blocage de sa position dans la base 2. La base 2 montre que les baïonnettes permettent d'assurer le blocage en position. Ladite position peut permettre une connexion électrique. Pour retirer la base 2 de son support, il est nécessaire de tourner d'un quart de tour pour retirer les baïonnettes de leur logement.

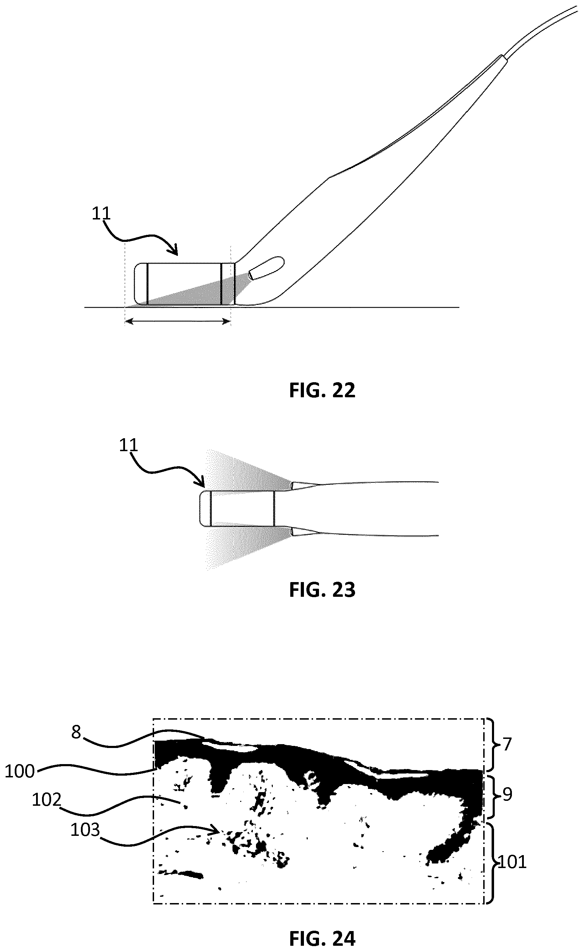

Les figures 22 et 23 permettent de montrer l'implantation des DEL sur le côté du dispositif 1. De cette manière, les DEL éclairent au moins toute la longueur de l'embout détachable 11 et les côtés de l'embout détachable 11.

Les dimensions de l'embout peuvent varier en fonction de l'utilisation. Pour la microdermabrasion, la longueur de l'embout détachable 11 peut être de l'ordre de 15 à 45 mm, de préférence 25 mm pour le visage et 35 mm pour le corps. Pour le massage, la longueur de l'embout détachable 11 peut être de l'ordre de 15 à 45 mm, de préférence 25 mm pour le visage et 35 mm pour le corps.

La figure 24 montre une coupe histologique de la peau d'un patient avant l'application du dispositif 1. Il est montré l'air ambiant 7, la couche cornée 8, l'épithélium 9, la membrane basale 100, le derme 101, le collagène 102 et la microvascularisation 103. Avant une première application, la peau est recouverte d'un épithélium régulier. Le derme 101 très superficiel est assez lâche. Il contient quelques vaisseaux et de rares cellules inflammatoires mononucléées.

Après deux séances de microdermabrasion, l'épiderme apparaît un peu modifié. Il est un peu épaissi avec une augmentation du nombre de kératinocytes d'environ 10 %. Dans le derme superficiel, on note un aspect plus fibreux au niveau du collagène le plus superficiel. Les vaisseaux sont plus nombreux de même que les cellules inflammatoires mononucléées.

Après quatre séances et massages, l'épiderme est plus épaissi d'environ 20 % par rapport à une zone non traitée. La densification du derme superficiel se maintient de même que la néovascularisation.

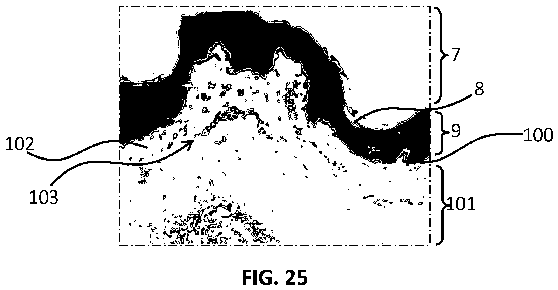

La figure 25 montre une coupe histologique de la peau d'un patient après quatre séances de microdermabrasion et de massage avec une crème. Il est également montré l'air ambiant 7, la couche cornée 8, l'épithélium 9, la membrane basale 100, le derme 101, le collagène 102 et la microvascularisation 103. L'épiderme apparaît augmenté d'épaisseur. Elle est appréciée à 25 %. Le collagène superficiel apparaît aussi plus dense. La microvascularisation persiste.

La microdermabrasion entraîne un épaississement de l'épiderme avec fibrose inflammatoire du derme superficiel. Ces aspects sont majorés avec le nombre de séances.

L'application de la crème avec massage amplifie les effets similaires du traitement.

Il a été remarqué que les peaux sensibles peuvent être sujettes à une légère sécheresse cutanée dans les trois jours qui suivent le traitement. Les pores sont resserrés et la peau uniformisée, ces effets perdurent jusqu'à trois semaines.

L'exemple suivant concerne le protocole d'utilisation du procédé de microdermabrasion.

### Pour le visage :

La patiente est en position allongée (ou légèrement relevée). La patiente est démaquillée ce qui permet de retirer toutes crèmes. La peau doit être la plus pure possible pour avoir le bénéfice maximal de la phase de microdermabrasion. Le visage est ensuite lavé (il n'y a pas de savon préconisé, mais il est recommandé un savon non allergisant, de pH neutre) et rincé. La peau ne doit absolument plus être humide. Une peau bien sèche assure une efficacité optimale de la microdermabrasion.

Une première étape du procédé est donc une phase de préparation de la peau, de nettoyage et séchage. Cette étape peut prendre trois à quatre minutes.

Concernant l'étape de microdermabrasion.

Son objectif est de modeler la peau afin d'améliorer le grain de la peau, atténuer les ridules, atténuer les vergetures, anticiper les effets du temps sur la peau, ralentir l'apparition d'imperfections et ralentir l'apparition d'irrégularités liées au temps par un lissage de la peau.

Dans un exemple de réalisation, l'étape de microdermabrasion est associée à une onde lumineuse (4 secondes pour chaque couleur, en rotation, jaune - orange - rouge)

Le dispositif est disposé à côté de la patiente. L'étape de la microdermabrasion consiste à prendre le dispositif comme un crayon, en faisant attention à ne pas obstruer les LED avec les doigts. Il est nécessaire d'installer au préalable un embout détachable sur le dispositif.

Ensuite, il est nécessaire de mettre le dispositif en mode microdermabrasion et de procéder à la microdermabrasion. Pour le visage, il est possible de procédé dans l'ordre suivant : 1/ la joue droite, 2/ la joue gauche, 3/ la surface en dedans des sillons nasogeniens (menton, lèvre inférieure, lèvre supérieure), 4/ nez et 5/ Front. Il ne faut pas passer sur les yeux et les paupières ni sur la partie rouge des lèvres.

Dans un autre exemple, l'élément abrasif 112 présente 5 facettes. C'est la facette du dessous qui agit lors de la mise en fonction en mode microdermabrasion. Ainsi, par un système de rotation on peut tourner l'embout détachable 11 et choisir une autre facette de l'élément abrasif 112 de traitement : chaque facette correspond environ à une zone traitée de 50 à 150 cm2. Pour le visage, chaque facette sera utilisée pour une zone : on traite la joue droite, puis on tourne l'embout et on utilise une nouvelle facette de microdermabrasion pour la joue gauche, puis on tourne l'embout et on utilise une nouvelle facette pour l'ensemble menton/ lèvre blanche inférieure et lèvre supérieure (en évitant la zone rouge de la lèvre), ainsi de suite pour les autres zones (le visage est divisé en 5 zones, et chaque zone sera traitée par une des facettes de l'embout).

Pour appliquer l'embout détachable sur la peau, il faut juste le poser ce qui permet ainsi le frottement inoffensif (l'abrasion s'arrête automatiquement quand la pression est plus forte, du fait de la souplesse de la peau qui se met à vibrer). Le geste peut être de faire des cercles dans le sens des aiguilles d'une montre, on traite ainsi à chaque fois un "disque de trois cm de diamètre", douze passages par disque, vitesse : une seconde par tour (on fait un rond en une seconde).

Il est fait douze ronds sur place (disques) de trois cm de diamètre dans le sens des aiguilles d'une montre, à la vitesse d'un rond par seconde environ. Autrement dit, chaque couleur dure 4 secondes, ainsi un cycle de couleur (Jaune, orange rouge) dure 12 secondes (puis un autre cycle Jaune, orange, rouge recommence), l'utilisateur fait environ 4 ronds au même endroit pour chaque couleur, ce qui donne 12 ronds pour un même disque en 12 secondes. Dans tous les cas, à la fin d'un cycle de couleur, le manipulateur passe au traitement de la peau voisine en traitant un disque sur la zone adjacente.

Par exemple par joue il est prévu dix à douze disques soit environ 120 secondes (2 minutes), pour le menton, lèvres inférieure et supérieure = cinq disques soit environ une minute, le nez = cinq disques soit environ une minute et le front = dix à douze disques soit environ 120 secondes (2 minutes).

L'étape du procédé de la microdermabrasion pour un visage est de huit minutes environ.

Une variante au niveau technique est (au lieu de traiter des disques) de faire des allers-retours sur une zone linéaire de 4 cm de long, à la largeur de l'embout détachable 112, chaque aller (ou retour) durant environ une seconde, soit deux aller retour par couleur, soit six aller retour par cycle de couleur sur une même zone, et on change de zone à la fin d'un cycle de couleur (ce qui donne six aller retour, soit douze allers par zone traitée).

Une fois que l'utilisateur a terminé de traiter une des grandes zones du visage (par ex la joue droite), l'utilisateur tourne l'embout détachable et positionne alors une facette neuve. L'utilisateur procédera de même pour la joue gauche, et pour les autres zones.

Suite à la microdermabrasion, il est réalisé une étape de nettoyage qui permet d'enlever toutes les impuretés et peaux mortes ou imperfections qui ont été lissées par le dispositif. Il est possible de réaliser ce nettoyage en passant une lingette, ce qui prend une minute environ.

L'étape de massage, correspondant à la phase de massage par l'élément déformable permet d'améliorer la qualité de la peau en termes d'épiderme, de vascularisation et de production de fibres collagène. Cette étape permet une meilleure imprégnation de la crème sur la peau purifiée, lissée et mise à nue par la microdermabrasion.

L'étape de massage consiste à appliquer une crème régénératrice, apaisante, astringente. Cette étape consiste à appliquer la crème sur une zone du visage, ne pas hésiter à en rajouter si la sensation de glisse n'est pas suffisante.

Il est nécessaire de passer en mode "massage". Il est nécessaire d'enlever élément abrasif et de placer l'élément déformable sur le visage avec le même ordre de passage que pour l'étape de microdermabrasion.

D'un point de vue de technique, c'est la même que pour la phase de microdermabrasion sauf que la durée est plus longue (par exemple doublée) en fonction du type de soin réalisé (ex : de 8 à 16 minutes en fonction de la durée du soin pour un visage). Puis un Nettoyage complet est fait (lavage au savon, en conseillant un savon neutre non allergisant), puis rinçage et séchage, ce qui peut prendre 3 à 4 minutes.

Une variante au procédé consiste à ne traiter qu'une zone du visage (ex : menton, lèvres supérieures et inférieures).

Pour le reste du corps, le procédé est le même avec les variables suivantes :
- on utilisera un embout « corps » à grain plus gros (180 g/m2) (contre 400 g/m2 pour le visage) ;
- le nombre de passage plus importants, par exemple, deux cycles de couleur, soit 24 secondes de traitement par disque, sauf pour les zones plus sensibles (mains, intérieur des cuisses, autour des aréoles) pour lesquelles on peut rester à 12 secondes soit un seul cycle de couleur par disque traité ;
- la surface traitée (visage entier = entre 200 et 300 cm2) = pour le corps, avec l'embout « corps » on peut traiter jusqu'à 400 cm2, mais si la surface traitée est plus grande, il faudra prendre un second embout.

### Nomenclature

1 dispositif
   11 embout détachable
      111 élément protecteur
      112 élément abrasif
      113 élément déformable
      114 centre rigide
   12 support d'embout
   13 moteur
   14 interrupteur
   15 capot
   16 coque
   17 câble
2 base
   21 interface
   22 moyen de connexion
   3 plateau d'accessoires
   4 sortie d'air
   5 zone de traitement
   6 transformateur
   7 air ambiant
   8 couche cornée
   9 épithélium
   100 membrane basale
   101 derme
   102 collagène
   103 microvascularisation
      A axe longitudinal de l'embout détachable
      B axe longitudinal du dispositif
      A angle entre l'axe longitudinal de l'embout détachable et l'axe longitudinal du dispositif

## Revendications

1. Dispositif (1) pour la microdermabrasion comprenant :
- un embout détachable (11) comprenant au moins un élément abrasif (112), ledit élément abrasif (112) étant prévu pour être appliqué sur la surface de la peau,
- un corps comportant un moyen vibrant, ledit moyen vibrant étant adapté pour la mise en vibration du corps ou de l'embout détachable (11),
- un support d'embout (12) fixé au corps, l'embout détachable (11) comprenant un élément déformable (113) positionné entre l'élément abrasif (112) et le support d'embout (12), ledit embout détachable (11) comprend un élément protecteur (111) ;
**caractérisé en ce que** l'élément protecteur (111) est adapté à protéger le patient de l'application de l'embout détachable (11) sur sa peau, ledit élément protecteur (111) étant positionné à l'extrémité de l'embout détachable et de la forme d'une demi-sphère ou d'une demi-sphère aplatie.

2. Dispositif (1) selon la revendication 1, **caractérisé en ce que** l'élément déformable (113) est en élastomère dont la mesure de l'élastomère est comprise entre 10 et 30, selon l'échelle de dureté Shore A.

3. Dispositif (1) selon la revendication 1, **caractérisé en ce que** dans un plan de coupe au dispositif (1) contenant l'axe longitudinal du corps et l'axe longitudinal du support d'embout (12), l'axe longitudinal du support d'embout (12) et l'axe longitudinal du corps forment un angle (α) de l'ordre de 90° à 270°, de préférence, de 135°.

4. Dispositif (1) selon la revendication 3, **caractérisé en ce que** le dispositif (1) comprend une liaison pivot entre le support d'embout (12) et le corps (16) du dispositif, l'axe de ladite liaison pivot étant contenu dans un plan orthogonal à l'axe longitudinal du corps (16) et passant par un point d'intersection de l'axe longitudinal du support d'embout (12) avec de l'axe longitudinal du corps (16).

5. Dispositif (1) selon l'une des revendications 1 à 4, **caractérisé en ce que** le corps de l'élément déformable (113) est de forme d'un ovale, d'un triangle, d'un carré, d'un polygone d'au moins cinq côtés ou d'un cercle, dont son centre est troué pour s'adapter au support d'embout (12).

6. Dispositif (1) selon l'une des revendications 1 à 5, **caractérisé en ce que** le moyen vibrant comprend un moteur (13) entraînant en rotation un élément comportant un balourd.

7. Dispositif (1) selon l'une des revendications 1 à 6, **caractérisé en ce que** ledit dispositif comprend des moyens pour faire varier la vitesse de rotation du moteur entre 0 tr/min et 12 000 tr/min.

8. Dispositif (1) selon l'une des revendications 1 à 7, **caractérisé en ce que** le dispositif (1) comprend au moins des moyens d'éclairage, notamment une diode électroluminescente dont sa longueur d'onde est comprise entre 400 et 900 nm et entre sa fluence comprise entre 3 et 4 J/cm².

9. Dispositif (1) selon l'une des revendications 1 à 8, **caractérisé en ce que** l'élément déformable (113) comprend une cavité agencée pour recevoir un volume d'une matière crémeuse, tel que de la crème adoucissante.

10. Dispositif (1) selon l'une des revendications 1 à 9, **caractérisé en ce que** le dispositif comprend des moyens d'étanchéité.

11. Dispositif (1) selon l'une des revendications 1 à 10, **caractérisé en ce que** l'élément abrasif (112) est un ruban a usage unique agencé pour être positionné autour de l'élément déformable (113) et une partie dudit ruban est détachable pour permettre le retrait de l'élément abrasif (113).

12. Dispositif (1) selon l'une des revendications 1 à 11, **caractérisé en ce que** l'embout détachable (11) est mobile en rotation autour de l'axe longitudinal du support d'embout, et l'embout détachable (11) est entraîné en rotation par un moyen rotatif.

13. Procédé de microdermabrasion pour des soins esthétiques non thérapeutique comprenant les étapes suivantes :
- a) mise en place d'un embout détachable (11) sur le dispositif (1) objet de l'invention selon l'une des revendications 1 à 12,
- b) mise en vibration de l'élément abrasif (112) sur la surface de la peau, pendant une durée de 1 à 30 minutes pour une surface de l'ordre de 50 à 300 cm².

14. Procédé selon la revendication 13, **caractérisé en ce qu'**il comprend une étape c), après l'étape b), enlèvement de l'élément abrasif (112) et application d'une crème adoucissante sur la surface de la peau pendant une durée de 2 à 60 minutes.

15. Procédé selon la revendication 14, **caractérisé en ce qu'**il comprend une étape d) préalable à l'étape a), de retrait du l'élément abrasif (112) d'un sac étanche.

## Patentansprüche

1. Mikrodermabrasionsvorrichtung (1), umfassend:
- ein abnehmbares Endstück (11), mindestens ein abrasives Element (112) umfassend, wobei das abrasive Element (112) vorgesehen ist, auf der Hautoberfläche angewandt zu werden,
- einen Körper, ein Vibrationsmittel enthaltend, wobei das Vibrationsmittel zum Versetzen des Körpers oder des abnehmbaren Endstücks (11) in Vibration eingerichtet ist,
- einen Endstückträger (12), der am Körper befestigt ist,
wobei das abnehmbare Endstück (11) ein verformbares Element (113) umfasst, das zwischen dem abrasiven Element (112) und dem Endstückträger (12) positioniert ist, wobei das abnehmbare Endstück (11) ein Schutzelement (111) umfasst; **dadurch gekennzeichnet, dass** das Schutzelement (111) eingerichtet ist, den Patienten vor der Anwendung des abnehmbaren Endstücks (11) auf seiner Haut zu schützen, wobei das Schutzelement (111) am Ende des abnehmbaren Endstücks positioniert und von der Form einer Halbkugel oder abgeflachten Halbkugel ist.

2. Vorrichtung (1) nach Anspruch 1, **dadurch gekennzeichnet, dass** das verformbare Element (113) aus einem Elastomer ist, dessen Elastomermaß nach der Härteskala Shore A zwischen 10 und 30 liegt.

3. Vorrichtung (1) nach Anspruch 1, **dadurch gekennzeichnet, dass** in einer Schnittebene der Vorrichtung (1), die die Längsachse des Körpers und die Längsachse des Endstückträgers (12) enthält, die Längsachse des Endstückträgers (12) und die Längsachse des Körpers einen Winkel (α) der Größenordnung von 90° bis 270°, vorzugsweise von 135°, bilden.

4. Vorrichtung (1) nach Anspruch 3, **dadurch gekennzeichnet, dass** die Vorrichtung (1) eine Schwenkverbindung zwischen dem Endstückträger (12) und dem Körper (16) der Vorrichtung umfasst, wobei die Achse der Schwenkverbindung in einer orthogonalen Ebene zur Längsachse des Körpers (16) enthalten ist und durch einen Schnittpunkt der Längsachse des Endstückträgers (12) mit der Längsachse des Körpers (16) verläuft.

5. Vorrichtung (1) nach einem der Ansprüche 1 bis 4, **dadurch gekennzeichnet, dass** der Körper des verformbaren Elements (113) von der Form eines Ovals, eines Dreiecks, eines Quadrats, eines Polygons mit mindestens fünf Seiten oder eines Kreises ist, dessen Zentrum gelocht ist, um sich an den Endstückträger (12) anzupassen.

6. Vorrichtung (1) nach einem der Ansprüche 1 bis 5, **dadurch gekennzeichnet, dass** das Vibrationsmittel einen Motor (13) umfasst, der ein Unwuchtelement in Rotation versetzt.

7. Vorrichtung (1) nach einem der Ansprüche 1 bis 6, **dadurch gekennzeichnet, dass** die Vorrichtung Mittel zum Variieren einer Drehzahl des Motors zwischen 0 U/min und 12.000 U/min umfasst.

8. Vorrichtung (1) nach einem der Ansprüche 1 bis 7, **dadurch gekennzeichnet, dass** die Vorrichtung (1) mindestens Leuchtmittel umfasst, insbesondere eine Leuchtdiode, deren Wellenlänge zwischen 400 und 900 nm liegt und deren Fluenz zwischen 3 und 4 J/cm² liegt.

9. Vorrichtung (1) nach einem der Ansprüche 1 bis 8, **dadurch gekennzeichnet, dass** das verformbare Element (113) einen Hohlraum umfasst, der zur Aufnahme eines Volumens eines cremigen Stoffes wie etwa einer lindernden Creme angeordnet ist.

10. Vorrichtung (1) nach einem der Ansprüche 1 bis 9, **dadurch gekennzeichnet, dass** die Vorrichtung Dichtmittel umfasst.

11. Vorrichtung (1) nach einem der Ansprüche 1 bis 10, **dadurch gekennzeichnet, dass** das abrasive Element (112) ein Band für Einmalgebrauch ist, das angeordnet ist, um um das verformbare Element (113) positioniert zu werden, und ein Teil des Bandes abnehmbar ist, um die Entnahme des abrasiven Elements (113) zu gestatten.

12. Vorrichtung (1) nach einem der Ansprüche 1 bis 11, **dadurch gekennzeichnet, dass** das abnehmbare Endstück (11) um die Längsachse des Endstückträgers drehbeweglich ist und das abnehmbare Endstück (11) durch ein Rotationsmittel in Rotation versetzt wird.

13. Mikrodermabrasionsverfahren für die nicht therapeutische Schönheitspflege, umfassend die folgenden Schritte:
- a) Aufsetzen eines abnehmbaren Endstücks (11) auf die Vorrichtung (1), die nach einem der Ansprüche 1 bis 12 Gegenstand der Erfindung ist,
- b) Versetzen des abrasiven Elements (112) in Vibration auf der Hautoberfläche während einer Dauer von 1 bis 30 Minuten für eine Oberfläche der Größenordnung von 50 bis 300 cm².

14. Verfahren nach Anspruch 13, **dadurch gekennzeichnet, dass** es nach dem Schritt b) einen Schritt c), Entfernung des abrasiven Elements (112) und Auftragen einer lindernden Creme auf die Hautoberfläche während einer Dauer von 2 bis 60 Minuten, umfasst.

15. Verfahren nach Anspruch 14, **dadurch gekennzeichnet, dass** es vor dem Schritt a) einen Schritt d) der Entnahme des abrasiven Elements (112) aus einem dichten Beutel umfasst.

## Claims

1. A device (1) for microdermabrasion comprising:
- a detachable tip (11) including at least one abrasive element (112), said abrasive element (112) being provided such as to be applied onto the surface of the skin,
- a body comprising a vibrating means, said vibrating means being capable of making the detachable tip (11) vibrate,
- a tip holder (12) that is securely attached to the body.
**characterized in that** the detachable tip (11) comprises a deformable element (113) positioned between the abrasive element (112) and the tip holder (12), said detachable tip (11) comprising a protective element (111) positioned at the end of the detachable tip and said protective element (111) being in the shape of a half-sphere or of a flattened half-sphere.

2. The device (1) as claimed in claim 1, **characterized in that** the deformable element (113) is made of elastomer, the measurement of which is between 10 and 30, as claimed in the Shore A hardness scale.

3. The device (1) as claimed in claim 1, **characterized in that** in a sectional plane to the device (1) containing the longitudinal axis of the body and the longitudinal axis of the tip holder (12), the longitudinal axis of the tip holder (12) and the longitudinal axis of the body form an angle (α) in the order of 90° to 270°, preferably 135°.

4. The device (1) as claimed in claim 3, **characterized in that** the device (1) comprises a pivot connection between the tip holder (12) and the body (16) of the device, the axis of said pivot connection being contained in a plane orthogonal to the longitudinal axis of the body (16) and passing through a point of intersection of the longitudinal axis of the tip holder (12) with the longitudinal axis of the body (16).

5. The device (1) as claimed in any one of the claims 1 to 4, **characterized in that** the body of the deformable element (113) is shaped as an oval, a triangle, a square, a polygon of at least five sides or a circle, having a hole in the center into which the tip holder can be adapted (12).

6. The device (1) as claimed in any one of claims 1 to 5, **characterized in that** the vibrating means comprises a motor (13) driving in rotation an element equipped with an unbalancing mass.

7. The device (1) as claimed in any one of claims 1 to 6, **characterized in that** said device comprises means for varying the rotation speed of the motor between 0 rpm and 12,000 rpm.

8. The device (1) as claimed in any one of claims 1 to 7, **characterized in that** the device (1) comprises at least lighting means, notably a light emitting diode whose wavelength is between 400 and 900 nm and whose fluence is between 3 and 4 J/cm².

9. The device (1) as claimed in any one of claims 1 to 8, **characterized in that** the deformable element (113) comprises a cavity arranged to receive a volume of a creamy material, such as a soothing cream.

10. The device (1) as claimed in any one of claims 1 to 9, **characterized in that** the device comprises sealing means.

11. The device (1) as claimed in any one of claims 1 to 10, **characterized in that** the abrasive member (112) is a single use tape arranged to be positioned around the deformable element (113) and a part of said tape is detachable to allow the abrasive element (113) to be removed.

12. The device (1) as claimed in any one of claims 1 to 11, **characterized in that** the detachable tip (11) is rotatably mobile about the longitudinal axis of the tip holder, and the detachable tip (11) is driven in rotation by a rotating means.

13. A microdermabrasion method for non-therapeutic cosmetic treatments comprising the following steps:
- a) placement of a detachable tip (11) on the device (1) as claimed in the invention as claimed in any one of claims 1 to 12,
- b) vibration of the abrasive element (112) on the surface of the skin, for a duration of 1 to 30 minutes over a surface area in the order of 50 to 300 cm².

14. The method as claimed in claim 13, **characterized in that** it comprises a step c), after step b), involving the removal of the abrasive element (112) and the application a soothing cream on the surface of the skin for a period of 2 to 60 minutes.

15. The method as claimed in claim 14, **characterized in that** it comprises a step d) prior to step a), involving the removal of the abrasive member (112) from a sealed bag.
